Europäisches Patentamt

(19) European Patent Office

Office européen des brevets

(11) Publication number: **0 140 004**
**B1**

(12) **EUROPEAN PATENT SPECIFICATION**

(45) Date of publication of the patent specification:
**29.10.86**

(21) Application number: **84110208.0**

(22) Date of filing: **28.08.84**

(51) Int. Cl.⁴: **C 12 N 9/02, C 12 Q 1/00**

(54) Bilirubin-specific enzymes and their use in analytical elements and methods.

(30) Priority: **01.09.83 US 528486**

(43) Date of publication of application:
**08.05.85 Bulletin 85/19**

(45) Publication of the grant of the patent:
**29.10.86 Bulletin 86/44**

(84) Designated Contracting States:
**CH DE FR GB IT LI SE**

(56) References cited:
**EP - A - 0 005 637**
**EP - A - 0 114 381**
**GB - A - 2 115 926**
**US - A - 4 069 017**

(73) Proprietor: **EASTMAN KODAK COMPANY, 343 State Street, Rochester New York 14650 (US)**

(72) Inventor: **Wu, Tai-Wing, 72 Andiron Lane, Rochester New York 14612 (US)**

(74) Representative: **Brandes, Jürgen, Dr.rer.nat. et al, Thierschstrasse 8, D-8000 München 22 (DE)**

# Description

This invention relates to enzymes having specific bilirubin degrading activity, and to their use in analytical elements and methods. It particularly relates to bilirubin-specific enzymes useful in analytical determinations of biological fluids.

Bilirubin is a yellow substance which is formed in the blood by degradation of hemoglobin, and is the principal pigment of bile manufactured in the liver. It has been estimated that approximately 200-230 milligrams of bilirubin and its derivatives are formed each day in a healthy human adult by the degradation of hemoglobin within the liver, spleen and bone marrow.

In human body fluids such as bile and serum, bilirubin exists in two different forms, these forms commonly being referred to in the clinical literature as conjugated bilirubin, $B_c$, and unconjugated bilirubin, $B_u$. The total bilirubin content $B_T$ represents the sum of $B_u$ and $B_c$.

The diagnostic significance of bilirubin is well established. For example, an excessive amount of bilirubin within the human body, referred to as jaundice, is recognized as evidence of a variety of disease conditions, particularly diseases of the liver. In addition, jaundice often occurs in new born infants whose liver is slow to begin normal function. Thus, to facilitate early diagnosis of certain disease states and/or to actively reduce bilirubin levels in samples to be tested, a bilirubin specific enzyme would be very useful.

Enzymes shown to be specific for bilirubin have been partially purified and characterized from certain fungi, as described in U.S. Patent 4,211,844 (issued July 8, 1980) and in the literature article by Mura and Tanaka, «A New Enzyme 'Bilirubin Oxidase' Produced by *Myrothecium verrucaria* MT-1», Agric. Biol. Chem., *45*(10), pp. 2383-2384 (1981). However, a problem with the enzymes obtained from such sources, particularly from the mushrooms described in the noted patent, is that they have rather low specific activity for bilirubin. Because of this low specific activity, larger quantities of enzymes are required to provide acceptable assay results. Furthermore, the fungi from which those enzymes are obtained are not always readily available in large quantities except at high expense.

This invention provides a solution for the described problem with known bilirubin-specific enzymes. This invention provides an enzyme and assay reagents which are highly specific to bilirubin and which contain enzymes obtained from relatively inexpensive sources.

The present invention provides a bilirubin specific enzyme characterized in that it is derived from a plant of the group of families consisting of Solanaceae, Musaceae and Liliaceae which, in the presence of a bilirubin-containing aqueous liquid, degrades unconjugated bilirubin at a pH in the range of from 6 to 10, conjugated bilirubin at a pH in the range of from 2 to 11, and both forms of bilirubin at a temperature in the range of from 20 to 50° C.

The enzyme can be used to degrade bilirubin (conjugated, unconjugated or a mixture of both forms) in an aqueous liquid, e.g. whole blood or serum, when bilirubin is present in a detrimental amount. The enzyme can also be included in an analytical reagent for the assay or detection of an analyte other than bilirubin in an aqueous liquid sample containing bilirubin. Such an analytical reagent comprises an interactive reagent for the analyte and the bilirubin-specific enzyme according to this invention. The enzyme thereby degrades bilirubin and reduces its interference with the assay.

The enzyme is useful in a dry analytical element for the assay of an aqueous liquid (i.e. the determination or detection of an analyte). In a preferred embodiment of this invention, this element includes a support and a reaction zone containing the enzyme and the interactive reagent.

Further still, this invention provides a method for the degradation of bilirubin in an aqueous liquid. This method is characterized by treating the liquid with the bilirubin-specific enzyme of this invention to degrade bilirubin.

The enzyme of this invention can also be used to assay for or to detect bilirubin (either $B_c$, $B_u$ or $B_T$) in an aqueous liquid by:

(a) treating a sample of the liquid with the bilirubin-specific enzyme according to this invention to interact bilirubin with the enzyme and to produce a detectable change; and

(b) detecting the change produced in step (a).

In another embodiment of this invention, a method for the assay or detection of an analyte other than bilirubin in an aqueous liquid containing bilirubin, bilirubin being an interferent, comprises the steps of:

(a) treating a sample of the liquid with an interactive reagent for the analyte to produce a detectable change;

(b) prior to or during step (a), treating the sample with the bilirubin-specific enzyme according to this invention, thereby degrading bilirubin and reducing its potential for interference with the detectable change produced in step (a); and

(c) detecting the change produced in step (a).

It has been found that the specificity of the enzyme of this invention for bilirubin is up to several times higher than the specificity of the known fungal enzyme described in U.S. Patent 4,211,844 noted above. Furthermore, the plants of these families, e.g. eggplants, tomatoes, potatoes, bananas, onions and garlic, are often much more readily available as enzyme sources and at lower cost than mushrooms.

A valuable property of the enzymes of this invention is their specific activity on bilirubin. Thus, when an enzyme of this invention is incubated with biliverdin or hemoglobin, both of which are highly colored and chemically similar to bilirubin, no visible change occurs (see Example 4 below). This indicates enzyme inactivity on substances closely related to bilirubin and specificity for bilirubin (both $B_c$ and $B_u$).

The bilirubin-specific enzymes of this invention can be extracted from plants of the families Solanaceae, Musaceae and Liliaceae. Examples of plants of the Solanaceae family are plants from the genera *Solanum, Capsicum* and *Lycopersicum*. Useful species of such plants include, but are not limited to, eggplants (*Solanum melongena*), sweet peppers (*Capsicum grossum*), tomatoes (*Lycopersicum esculentum*) and Irish potatoes (*Solanum tuberosum*). Examples of plants of the Musaceae family are plants from the *Musa* genus and include, but are not limited to, bananas and related plants (*Musa paradisiaca, Musa sapientum* and *Musa acuminata*). Examples of plants of the Liliaceae family are plants from the *Allium* genus and include, but are not limited to, onions (*Allium cepa*), garlic (*Allium sativum*), chives (*Allium schoenoprasum*), leeks (*Allium porrum*) and wild onions (*Allium cernuum*).

The enzymes of of this invention can be extracted from the desired plants by any suitable extraction method. Two such methods are described in detail in U.S. Patent 4,211,844 noted above. Useful extraction procedures are described below in Examples 1, 5, 8 and 10.

Bilirubin exhibits a characteristic absorption peak ($\lambda$max) at about 440 nanometers (nm) of the electromagnetic spectrum. When the enzyme of this invention degrades bilirubin ($B_c$ or $B_u$) in a liquid sample under suitable pH and temperature conditions, the absorbance (or absorption density) at $\lambda$max decreases. This density decrease is a detectable change and can be monitored against a reference sample which lacks bilirubin. In this manner, an aqueous liquid sample can be assayed for bilirubin.

One use of the enzyme of this invention is to degrade bilirubin in a biological fluid, e.g. whole blood, when bilirubin is present in that fluid in an excessive amount. This can be done by treating the fluid with the enzyme, e.g. by passing the fluid through a filter device (either implanted in a patient or *ex vivo*) containing the enzyme. A bilirubin-binding material, e.g. albumin or a mordant, such as that described in U.S. Patent 4,338,095 (issued July 6, 1982 to Wu), can be used in combination with the enzyme to remove any undegraded bilirubin or the products of bilirubin degradation.

Another use of the enzyme of this invention is for the assay or detection of bilirubin (either $B_c$, $B_u$ or $B_T$) in an aqueous liquid, such as a biological fluid (e.g. whole blood, plasma, sera, lymph, bile urine, spinal fluid, sputum, sweat and the like as well as stool secretions of humans or animals). Treatment of the liquid containing bilirubin with the enzyme produces a detectable change which corresponds to the amount of bilirubin (either $B_c$, $B_u$ or $B_T$). $B_c$ can be detected by carrying out the assay at a pH less than 6. $B_T$ can be detected by carrying out the assay at a pH between 6 and 10. $B_u$ can be detected by subtracting $B_c$ from $B_T$.

Still another use of the enzyme of this invention is to reduce bilirubin interference in assays for analytes other than bilirubin. Such assays are usually achieved with analytical reagents and elements containing the enzyme and interactive reagents which, when they interact with the analyte, produce a detectable change of some type (e.g. colorimetric, potentiometric, etc.). Any suitable interactive reagent can be used with the bilirubin-specific enzyme in the analytical reagents. Examples of interactive reagents include those useful in enzyme-linked hydrogen peroxide detection systems, enzyme-linked NAD-NADH detection systems, redox reactions, hydrolysis reactions and others known in the clinical chemistry art.

The interactive reagent useful in an assay of an analyte other than bilirubin is a reagent or combination of reagents capable of physical, electrical, chemical or other interaction with the analyte of interest leading to a detectable change, for example, an absorbance shift or a change in absorption density, which can be related to the presence and/or amount of the analyte.

In this embodiment of reducing bilirubin interferences, a liquid sample is treated with the bilirubin-specific enzyme prior to, or at the same time as, the sample is treated with the interactive reagent. The enzyme reduces the potential of bilirubin interference in the assay for the analyte.

The enzyme of this invention is effective to interact with both conjugated and unconjugated forms of bilirubin within wide pH range. For unconjugated bilirubin, the enzyme is generally effective over a useful pH range of from 6 to 10; more effective in a range of from 6.5 to 8.5; and most effective in a range of from 7.0 to 7.5. For conjugated bilirubin, the enzyme is generally effective over a useful pH range of from 2 to 11; more effective in a range of from 3 to 9; and most effective in a range of from 6.0 to 8.0. By adjusting the assay pH, one can detect either or both forms of bilirubin as described above.

The enzyme is effective to interact with both forms of bilirubin in a useful temperature range of 20 to 50° C. However, it is particularly effective in a temperature range of from 25 to 45° C; and most effective in a range of from 30 to 37° C.

A buffer can also be present with the enzyme to maintain the pH within the pH range effective for bilirubin degradation. Phosphates, such as sodium phosphate, are suitable. However, a variety of other useful buffers are appropriate and are described, for example, by Good in *Biochemistry, 5*, 467 (1966).

The amounts of the various components of the analytical reagent may vary widely and may depend upon the bilirubin concentration of the liquid sample to be assayed. Generally, in solution or «wet» assays, the enzyme is used in an amount in the range of from 0.01 to 0.2, and preferably in the range of from 0.02 to 0.1, mg/dl. This assumes that each mg of enzyme has the minimum activity level for bilirubin (either $B_c$ or $B_u$) of at least 0.02 micromoles of bilirubin (either $B_c$ or $B_u$) per minute as determined in an aqueous liquid at a pH of 7.3 and a temperature of 37° C. When using an enzyme of higher activity, proportionately smaller amounts of the enzyme can be used.

When the analytical reagent of this invention is employed to eliminate or reduce bilirubin as an interferent in an assay, the interactive reagent which is employed must itself be non-interfering with respect to the enzyme.

The detectable change produced by the methods of this invention can be detected by a wide variety of means. For example, a suitable detection device capable of detecting a change in absorption density, a change in fluorescent or radioactive emission or a shift in the absorbance of a characteristic λmax can be employed.

The enzyme, analytical reagents and methods of this invention can be employed in either solution analytical techniques, sometimes called «wet chemistry» or dry analytical techniques, sometimes called «dry chemistry». A solution assay is carried out entirely in a liquid medium, and the enzyme or analytical reagent is employed as a liquid reagent. In such case, the enzyme or analytical reagent is mixed with a sample of the aqueous liquid to be assayed at an appropriate pH and temperature. This solution assay technique is described in more detail below in Example 2.

When employed in «dry chemistry» assays, the enzyme or analytical reagent can be incorporated into a reagent zone of a dry analytical element by imbibition, impregnation, coating or other suitable techniques. For example, the enzyme can be incorporated into a reagent layer of a dip-and-read fibrous test strip or a fibrous or non-fibrous multilayer element, such as that described in U.S. Patents 3,992,158 (issued November 16, 1976 to Przybylowicz et al), 4,042,335 (issued August 16, 1977 to Clément), 4,144,306 (issued March 13, 1979 to Figueras), 4,258,001 (issued March 24, 1981 to Pierce et al) and 4,292,272 (issued September 29, 1981 to Kitajima et al). The assay is then practiced by contacting (e.g. spotting) the element with the aqueous liquid sample to be assayed. Any detectable change which results is measured with appropriate apparatus. The enzyme or analytical reagent is present in such elements as a dried residue (e.g. a freeze-dried powder or dried residue of a coating solution).

When used in «dry chemistry» assays, the bilirubin-specific enzyme is generally present in an amount in the range of from 40 to 400 U/m², and preferably in an amount in the range of from 50 to 200 U/m².

The dry analytical elements of this invention generally have at least one absorbent reagent zone containing the enzyme or interactive reagent, or both. This zone can be self-supporting (i.e. composed of materials rigid enough to maintain its integrity), but preferably it is carried on a support. Such a support can be of any suitable dimensionally stable, and preferably, transparent (i.e. radiation transmissive) material which transmits electromagnetic radiation of a wavelength between about 200 and 900 nm. Useful support materials include polystyrenes, polyesters [e.g. poly(ethylene terephthalate)], polycarbonates, cellulose esters, etc. The element can have a plurality of zones, some or all containing reagents. These zones are in fluid contact with each other, meaning that fluids can pass between superposed regions of adjacent zones. Stated in another manner, fluid contact refers to the ability to transport components of a fluid between the zones in fluid contact. Preferably, the zones are separate coated layers, although one or more layers can be in a single zone of an element. Dry element formats and materials to make such are known and described, for example, in the patents noted above.

Any of the reagent zones of these elements can also act as an absorbent spreading zone. Additionally, or alternatively, the element can have one or more separate spreading zones. A spreading zone is typically a layer which can absorb a liquid sample. When the liquid sample is applied directly to the layer or provided to it from a layer or layers in fluid contact with it, the solvent or dispersion medium of the sample is distributed such that a uniform concentration of the sample is provided at the surface of the spreading layer facing the adjacent zone. Useful materials for preparing spreading zones are described, for example, in the patents noted above. Preferably, the spreading zone is an isotropically porous spreading layer as described in U.S. Patent 3,992,158, noted above.

A variety of different dry analytical elements, depending on the method of assay, can be prepared in accordance with the present invention. Elements can be configured in a variety of forms, including elongated tapes of any desired width, sheets or smaller chips.

The methods of this invention can be manual or automated. In general, the amount of bilirubin or other analyte in a liquid is assayed or detected by taking the element from a supply roll, chip packet or other source and physically contacting it with a sample of the liquid. Such contact can be accomplished in any suitable manner, e.g. dipping or immersing the element into the sample or, preferably, by spotting the element by hand or machine with a drop of the sample by pipette or another suitable dispensing means.

After sample application, the element is exposed to any conditioning, such as incubation, heating or the like, that may be desirable to quicken or otherwise facilitate obtaining any test result.

The analyte or bilirubin, if present, then reacts and produces a detectable change which is quantifiable by passing the element through a zone in which suitable apparatus for detection is provided.

Suitable detection means include the use of reflection or transmissive colorimetric spectrophotometry, fluorescence spectrophotometry, radiometry, chemiluminescence, enzyme labeling, measurement of enthalpy changes and the like.

The examples below further illustrate the invention. The following information is common to the examples.

Protein concentration was determined by the method of Warburg & Christian (*Biochem. Z.,* *310*:384, 1941) using a ratio of absorbances measured at 280 and 260 nm. Unless otherwise stated, all enzyme extraction steps were carried out at 0-4° C. All chemicals were reagent grade and were obtained from Eastman Kodak Co., Roches- ter, New York. Bilirubin ($B_u$) was obtained from Sigma Chemical Co., St. Louis, Missouri. Conjugated bilirubin was isolated from human bile according to the procedure described in U.S. Patent 4,311,665 (issued January 19, 1982 to Wu) and in an article by Wu et al in *Clin. Chem.*, *26*(9), pp. 1323-1335 (1980). A bilirubin ($B_u$) stock solution (100 mg/ml) was prepared by dissolving the weighed, solid material (prewet with about 100 µl of 0.1N NaOH) in 0.05 M sodium phosphate buffer (pH 7.45) in a 100 ml flask. This solution was kept at 0-4° C under a nitrogen-enriched atmosphere. A stock solution of conjugated bilirubin was similarly prepared and stored. A molar absorptivity of $55 \times 10^3$ 1/mole/cm at 440 nm was used for bilirubin (see method of Jacobsen & Wennberg, *Clin. Chem.*, *20*:783, 1974). Just prior to use, the bilirubin ($B_u$) stock solution was diluted with 0.05 M of the sodium phosphate buffer.

Bilirubin-containing solutions, buffered as stated above were incubated with an aliquot of the enzyme and the decrease in absorption density at λmax (440 nm) was monitored against a reference solution of identical composition but lacking bilirubin. The final volume of each reaction mixture was 1.01 ml. All assays were monitored at 22-25° C on a Perkin-Elmer Model 576 Spectrophotometer at 440 nm, unless otherwise stated.

*Example 1:*

*Extraction of Enzyme from Eggplants of the Solanaceae Family*

Eggplants were rinsed with distilled water, sliced and added to 3-4 volumes of ice-chilled 0.05 M sodium phosphate buffer (pH 7.45). The eggplant tissues were then homogenized with a conventional blender with 10-12 separate bursts, each burst lasting 10-15 seconds, interspersed with 30-second periods of ice-bath chilling. The resulting homogenate was filtered through 3 layers of cheesecloth. The brown filtrate was then centrifuged in a Beckman JC-21 centrifuge at 9,000 xg and 4° C for 20 minutes. The resulting pellet, exhibiting negligible bilirubin-specific activity, was discarded, and the supernatant was treated with an equal volume of dry ice-chilled acetone. The mixture was stirred in an ice bath containing both ordinary and dry ice. When the temperature of the solution reached −10° C, stirring was stopped and the solution was left to stand at 4° C for 20 minutes before it was centrifuged at 9,000 xg and 4° C for 30 minutes. The resulting pellets were resuspended in 1-2 volumes of 0.05 M sodium phosphate buffer (pH 7.45) and diluted with ice-chilled ethanol (1:1, V/V). The resulting mixture was centrifuged at 8,000 xg and 4° C for 15 minutes, whereupon it separated into three phases: (1) an upper clear liquid which exhibited most of the enzyme activity; (2) a cloudy interface exhibiting some activity; and (3) a pellet at the bottom of the centrifuge tube exhibiting no activity. Two repeated extractions of the cloudy interface using ethanol resulted in the release of more enzyme activity into the upper clear phase. The clear upper phase was dried down and resuspended in the buffer. It exhibited full enzyme activity for degrading bilirubin ($B_u$). Results obtained from enzyme extracted by this procedure from three separate batches of eggplant starting material are shown in Table I below. The enzyme can be further purified by conventional techniques such as affinity chromatography and electrofocusing.

*Table I*

| Purification Step | Protein | | | | Activity | | |
|---|---|---|---|---|---|---|---|
| | Eggplant Batch* | mg/ml | Total (mg) | % Recovery of Protein | Specific Activity** | Total Units | % Recovery of Activity |
| Crude Homogenate | 1 | 12.4 | 9293 | 100 | 0.0186 | 173.2 | 100 |
| | 2 | 9.4 | 7065 | 100 | 0.0245 | 173.1 | 100 |
| | 3 | 15.9 | 11888 | 100 | 0.0156 | 185.5 | 100 |
| Acetone Precipitation | 1 | 7.1 | 1065 | 11.5 | 0.2186 | 232.9 | 134.4 |
| Ethanol Extraction | 1 | 1.5 | 38.3 | 0.41 | 0.439 | 16.8 | 9.7 |
| | 2 | 1.53 | 38 | 0.54 | 0.483 | 18.5 | 10.6 |
| | 3 | 2.3 | 56.5 | 0.48 | 0.370 | 20.9 | 11.3 |

* The acetone-treated material was not assayed from batches 2 and 3. Those batches were carried directly to ethanol extraction.

** Activity was determined by calculating the number of µ moles of bilirubin ($B_u$) degraded per minute per mg, based on the rate of change at $A_{440}$ using a molar extinction coefficient of $59 \times 10^3$ at pH 7.45 and 25° C. A 2 mg/ml bilirubin ($B_u$) solution was used in activity tests.

The enzyme was solubilized in each of several alcohols (e.g. 1:1 v/v ethanol, propanol or butanol) to form a clear solution that was stable for storage over several days at 0-4° C.

Several other solubilization techniques were tried. The results are listed in Table II below.

Table II

Immediate Effect

| Solubilization Technique | Solubility | Specific Activity** | Long-Term Effects |
|---|---|---|---|
| Sodium deoxycholate detergent* (0.5-2%) | increased | little change | activity decayed overnight |
| Cetyltrimethylammonium bromide* detergent (0.5-1%) | not tested | greatly reduced | not tested |
| Bee venom phospholipase A*** (20 µg-1 mg) | unchanged | unchanged | not tested |
| Snake venom phospholipase A*** (20 µg-1 mg) | unchanged | unchanged | not tested |
| Bee venom phospholipase A*** (1 mg)+deoxycholate (1.5%) | increased | little change | activity decayed overnight |
| Heating 14 mg/ml enzyme to 78° C for 1 minute | activity remained in pellet | little change | great activity loss after few hours |

* Commercially available from Sigma Chemical Co. (St. Louis, Missouri)
** µ moles of bilirubin (B$_u$) consumed/min/mg of enzyme
*** Bee venom phospholipase A had 22,000 dl/mg solid; 1 unit hydrolizes 1 mole of L-α-phosphatidyl choline to L-α-lysophosphatidyl choline per minute at pH 8.5 and 85° C. The Snake venom (*Crotalux Terrificus terrificus*) phospholipase A had 200 U/mg solid. Both were obtained from Sigma Chemical Co. (St. Louis, Missouri)

The effect of temperature on the initial velocity of the enzyme-bilirubin (B$_u$) reaction was evaluated. The bilirubin (B$_u$) and enzyme concentrations were maintained at 2 mg/dl ($34.2 \times 10^{-6}$ M) and 0.09 mg/dl, respectively. The change in absorbance at 440 nm was monitored at various temperatures ranging from 25° C to 50° C.

The rate of bilirubin (B$_u$) degradation due to natural causes (e.g. oxidation) was subtracted from the rate of bilirubin (B$_u$) degradation caused by the enzyme. A gradual rise in reaction velocity was observed as the temperature was increased from 25° C to 37° C, followed by a great increase in the velocity as the temperature was increased from 40° C to 50° C. These results indicate that there is a change from one value of activation energy to another at 37-38° C.

The effect of NaCl concentration on the initial velocity of the enzyme-bilirubin (B$_u$) reaction was also evaluated. The rate of bilirubin (B$_u$) degradation was monitored as described in Example 2 below, except that the concentration of enzyme used was held constant at 0.085 mg protein. NaCl was added to aliquots of the bilirubin (B$_u$) solution (1.8 mg/dl, pH 7.4) to achieve final concentrations up to 1 M NaCl.

It was observed that the reaction velocity rose gradually with NaCl up to 0.1 M. Beyond this concentration, the velocity decreased steadily until, at 1.0 M NaCl, it was less than 50% of the velocity at 0.1 M NaCl.

*Example 2:*

*Bilirubin (B$_u$) Degradation in Solution Assay as a Function of Eggplant Enzyme Concentration*

Different amounts of the enzyme extract obtained from eggplants by the procedure described in Example 1 were added to separate liquid samples containing 2 mg/dl bilirubin (B$_u$) freshly prepared in 0.05 M sodium phosphate buffer (pH 7.45) at 25° C. The decrease in absorption density at λmax (440 nm) was monitored against a reference of identical composition but without bilirubin (B$_u$) using a conventional spectrophotometer. It was observed that the initial velocity of the degradation reaction varies linearly with the amount of enzyme used up to about 0.17 mg protein. At higher amounts, linearity was not observed.

*Example 3:*

*Bilirubin (B$_u$) Degradation in Solution Assay as a Function of Bilirubin (B$_u$) Concentration*

An aliquot containing 0.09 mg of enzyme obtained from eggplants by the procedure described in Example 1 was added to bilirubin (B$_u$)-containing liquid samples having concentrations of bilirubin (B$_u$) ranging from $2.8 \times 10^{-6}$ to $2 \times 10^{-5}$ moles/l. Test conditions were the same as those described in Example 2. The apparent extrapolated Michaelis Constant, Km, of the eggplant bilirubin-specific enzyme was determined to be $1 \times 10^{-4}$ M (i.e., 5.8 mg/dl) (see Lineweaver & Burk, *J.A.C.S., 56*, p. 658, 1934).

*Example 4:*

*Bilirubin Specificity of Eggplant Enzyme*

This example illustrates the specificity of the eggplant enzyme of this invention for bilirubin.

In the normal metabolism of heme in mammals, hemoglobin and biliverdin are known precursors of bilirubin. Assays were carried out in the following manner to see if the enzyme prepared in Example 1 would show any activity toward hemoglobin or biliverdin.

The eggplant enzyme of Example 1 was added to two solutions of hemoglobin (0.5 and 1.4 g/dl, respectively) and to two solutions of biliverdin (0.9 and 5 mg/dl, respectively) to achieve a final enzyme concentration of about 1 mg/dl. Repetitive spectral scans were made on each solution for a period of 60 minutes. The hemoglobin solutions were scanned at 420 ($\lambda$max), 540 and 576 nm; and the biliverdin solutions were scanned at 3890 ($\lambda$max) and 670 nm.

In each of the spectral scans, it was observed that the $\lambda$max was unchanged and that no change in absorption density occurred for periods of time up to one hour. These results indicate that the enzyme does not show any specificity for hemoglobin or biliverdin.

*Example 5:*

*Extraction of Enzyme from Bananas of the Musaceae Family*

The pulp of half of one banana was homogenized in five volumes of ice-chilled 0.05 M sodium phosphate buffer (pH 7.45), filtered through cheesecloth and centrifuged in a manner described for the extraction of eggplants in Example 1. The supernatant was immediately frozen at −5° C until its use.

*Example 6:*

*Bilirubin ($B_u$) Degradation in Solution Assay as a Function of Banana Enzyme Concentration*

Different amounts of the crude enzyme extract obtained from bananas by the procedure described in Example 5 were added to separate liquid samples containing 2.74 mg/dl bilirubin ($B_u$) freshly prepared in 0.05 M sodium phosphate buffer (pH 7.45) at 25° C. The decrease in absorption density at $\lambda$max (460 nm) was monitored against a reference of identical composition but without bilirubin ($B_u$) using a conventional spectrophotometer. It was observed that the initial velocity of the degradation reaction varies linearly with the amount of enzyme used up to about 200 µl (0.43 µg protein/µl).

*Example 7:*

*Bilirubin Degradation in Solution Assays as a Function of Bilirubin ($B_u$) Concentration*

A 200 µl aliquot containing about 86 µg of crude banana enzyme was added to bilirubin ($B_u$)-containing liquid samples having bilirubin ($B_u$) concentrations up to 4 mg/dl. Test conditions were the same as those described in Example 6. It was observed that the initial velocity of the crude banana enzyme varies linearly with substrate concentration. The range of apparent extrapolated Michaelis Constant, Km, was determined to be from $5 \times 10^{-5}$ to $1 \times 10^{-7}$ M.

*Example 8:*

*Extraction of Enzyme from Onions of the Liliaceae Family*

An onion was frozen at −70° C and subsequently thawed. The first 2 or 3 layers were peeled off and the remainder of the onion was sliced, minced, homogenized with five volumes of ice-chilled 0.05 M sodium phosphate buffer (pH 7), filtered through cheesecloth and centrifuged in a manner described for the extraction of eggplants in Example 1. The supernatant was separated into 5 ml aliquots and stored at 0-4° C until its use.

*Example 9:*

*Bilirubin ($B_u$) Degradation in Solution Assay as a Function of Onion Enzyme Concentration*

Different amounts of the enzyme extract obtained from onions by the procedure described in Example 8 were added to separate liquid samples containing 2 mg/dl bilirubin ($B_u$) freshly prepared in 0.05 M sodium phosphate buffer (pH 7) at 25° C. The decrease in absorption density at $\lambda$max (440 nm) was monitored against a reference of identical composition but without bilirubin ($B_u$) using a conventional spectrophotometer. It was observed that the initial velocity of the degradation reaction varies linearly with the amount of enzyme used up to about 100 µg protein. The range of apparent extrapolated Michaelis Constant, Km, was determined to be from $5 \times 10^{-5}$ to $1 \times 10^{-7}$ M.

*Example 10:*

*Extraction of Enzyme from Garlic of the Liliaceae Family and Bilirubin ($B_u$) Degradation by Enzyme*

Fresh garlic (11.9 g), with its skin peeled off, was homogenized for 15 seconds with a commercial food blender in 75 ml of 0.1 M phosphate buffer solution (pH 7.4). The resulting mixture was then placed in ice for 20-30 seconds. This procedure was repeated four times, after which the mixture was filtered through cheese cloth and centrifuged at 4000 rpm for 15 minutes at 0-4° C. The supernatant contained the bilirubin-specific enzyme.

The enzyme isolated as described above was evaluated for its activity for bilirubin ($B_u$) in the following manner.

A stock bilirubin ($B_u$) solution was prepared by dissolving 2.5 mg of unconjugated bilirubin (obtained from Sigma Chemical Co., St. Louis, Missouri) in 100 µl of 1 N NaOH. This was diluted to a total volume of 25 ml with 0.05 M phosphate buffer (pH 7.4) to yield a 10 mg/dl bilirubin ($B_u$) solution.

All test were made in 1 cm pathlength cuvettes and scanned on a conventional Perkin-Elmer spectrophotometer. The absorbance of each test sample was recorded at 450 nm at time 0 (time of mixing enzyme and bilirubin solution) and at 5 minutes after addition of enzyme to each cuvette. All reference samples (containing some enzyme concentration but no bilirubin) and test samples contained 1.3 ml of solution. The test samples contained the enzyme concentrations noted below in Table III. The range of apparent extrapolated Michaelis Constant, Km, was determined to be from $5 \times 10^{-7}$ to $1 \times 10^{-8}$ M.

*Table III*

| Test | Enzyme Concentration (ml of supernatant) | Bilirubin ($B_u$) Concentration (ml of stock solution) | $\Delta A/min$* at 450 nm |
|------|------------------------------------------|--------------------------------------------------------|---------------------------|
| 1 | 0.2 | 0.1 | 0.32 |
| 2 | 0.2 | 0.2 | 1.48 |
| 3 | 0.2 | 0.3 | 1.54 |
| 4 | 0.3 | 0.1 | 0.94 |
| 5 | 0.4 | 0.1 | 1.0 |
| 6 | 0.5 | 0.1 | 0.44 |

\* Rate of change in absorbance per minute at 450 nm as determined by subtracting the absorbance after 5 minutes from the absorbance at time 0, and dividing the difference by 5.

*Example 11:*

*Degradation of Bilirubin ($B_c$)*

The activity of the enzyme extracted from eggplants as described in Example 1 above was evaluated for $B_c$ in the following manner. The enzyme was suspended in either 0.1 M phosphate or 0.1 M Tris·HCl buffer at various pH values. Human bile isolated $B_c$ was used as the enzyme substrate at 2 mg/dl. These tests showed that the enzyme was active to degrade $B_c$ over a useful pH range of from 2 to 11 and had an optimum activity at 7.4.

**Claims**

1. A bilirubin-specific enzyme characterized in that it is derived from a plant of the group of families consisting of Solanaceae, Musaceae and Liliaceae which, in the presence of a bilirubin-containing aqueous liquid, degrades bilirubin ($B_u$) at a pH in the range of from 6 to 10, bilirubin ($B_c$) at a pH in the range of from 2 to 11, and both $B_u$ and $B_c$ at a temperature in the range of from 20 to 50° C.

2. An analytical reagent comprising an interactive reagent for the assay or detection of an analyte other than bilirubin in an aqueous liquid sample, the analytical reagent characterized in that it comprises a bilirubin-specific enzyme derived from a plant of the group of families consisting of Solanaceae, Musaceae and Liliaceae which, in the presence of a bilirubin-containing aqueous liquid, degrades bilirubin ($B_u$) at a pH in the range of from 6 to 10, bilirubin ($B_c$) at a pH in the range of from 2 to 11, and both $B_u$ and $B_c$ at a temperature in the range of from 20 to 50° C.

3. The analytical reagent as claimed in claim 2 which contains a buffer for maintaining the pH in the range of from 2 to 11.

4. A dry analytical element for the assay or detection of an analyte characterized in that it comprises an enzyme according to claim 1 or an analytical reagent according to claim 2 or 3.

5. The element as claimed in claim 4 comprising a support and a reagent zone containing the enzyme or analytical reagent.

6. The element as claimed in claim 4 or 5 comprising an absorbent spreading zone.

7. The element as claimed in any of claims 4 through 6 in which the enzyme is present in an amount of from 40 to 400 U/m².

8. A method for the degradation of bilirubin in an aqueous liquid, the method characterized by treating the liquid with a bilirubin-specific enzyme according to claim 1 to degrade bilirubin.

9. The method as claimed in claim 8 in which the aqueous liquid is whole blood.

10. A method for the assay or detection of bilirubin ($B_u$, $B_c$ or $B_T$) in an aqueous liquid, the method characterized by the steps of:

(a) treating a sample of the liquid with a bilirubin-specific enzyme according to claim 1 to interact bilirubin with the enzyme and to produce a detectable change, and

(b) detecting the change produced in step (a).

11. A method for the assay or detection of an analyte other than bilirubin in an aqueous liquid containing bilirubin, bilirubin being an interferent, the method characterized by the steps of:

(a) treating a sample of the liquid with an interactive reagent for the analyte to produce a detectable change,

(b) prior to or during step (a), treating the sample with a bilirubin-specific enzyme according to claim 1, thereby degrading bilirubin and reducing its potential for interference with the detectable change produced in step (a), and

(c) detecting the change produced in step (a).

12. The method as claimed in claim 10 or 11 in which the enzyme or interactive reagent is contained in a dry analytical element according to any of claims 4 through 7.

13. The invention as claimed in any of claims 1-12 in which the enzyme is derived from a plant of the *Solanum, Capsicum* or *Lycopersicum* genera of the family Solanaceae.

14. The invention as claimed in any of claims 1 through 12 in which the enzyme is derived from a plant of the *Musa* genus of the family Musaceae.

15. The invention as claimed in any of claims 1 through 12 in which the enzyme is derived from a plant of the *Allium* genus of the family Liliaceae.

16. The invention as claimed in any of claims 13 through 15 in which the enzyme is derived from *Solanum melongena, Musa sapientum, Allium cepa* or *Allium satiuum.*

## Patentansprüche

1. Bilirubin-spezifisches Enzym, dadurch gekennzeichnet, dass es von einer Pflanze aus einer der Familien der Solanaceaen, Musaceaen und Liliaceaen stammt, das in Gegenwart einer Bilirubin enthaltenden wässerigen Flüssigkeit, Bilirubin ($B_u$) bei einem pH-Wert von 6 bis 10, Bilirubin ($B_c$) bei einem pH-Wert von 2 bis 11 und sowohl $B_u$ als auch $B_c$ bei einer Temperatur von 20 bis 50° C abbaut.

2. Analytisches Reagens mit einem reaktiven Reagens für die Prüfung auf oder Bestimmung eines von Bilirubin verschiedenen Analyten in einer wässerigen Flüssigkeitsprobe, dadurch gekennzeichnet, dass das analytische Reagens ein Bilirubin-spezifisches Enzym enthält, das von einer Pflanze aus einer der Familien der Solanaceaen, Musaceaen und Liliaceaen stammt, das in Gegenwart einer Bilirubin enthaltenden wässerigen Flüssigkeit, Bilirubin ($B_u$) bei einem pH-Wert von 6 bis 10, Bilirubin ($B_c$) bei einem pH-Wert von 2 bis 11 und sowohl $B_u$ als auch $B_c$ bei einer Temperatur von 20 bis 50° C abbaut.

3. Analytisches Reagens nach Anspruch 2, dadurch gekennzeichnet, dass es einen Puffer für die Aufrechterhaltung des pH-Wertes im Bereich von 2 bis 11 enthält.

4. Trockenes analytisches Element für die Prüfung auf oder Bestimmung eines Analyten, dadurch gekennzeichnet, dass es ein Enzym nach Anspruch 1 oder ein analytisches Reagens nach Anspruch 2 oder 3 enthält.

5. Element nach Anspruch 4, dadurch gekennzeichnet, dass es einen Träger und eine Reagenszone mit dem Enzym oder analytischen Reagens aufweist.

6. Element nach Anspruch 4 oder 5, dadurch gekennzeichnet, dass es eine absorbierende Ausbreitzone aufweist.

7. Element nach einem der Ansprüche 4 bis 6, dadurch gekennzeichnet, dass es das Enzym in einer Menge von 40 bis 400 E/m² enthält.

8. Verfahren zum Abbau von Bilirubin in einer wässerigen Flüssigkeit, dadurch gekennzeichnet, dass man die Flüssigkeit zum Abbau von Bilirubin mit einem Bilirubin-spezifischen Enzym nach Anspruch 1 behandelt.

9. Verfahren nach Anspruch 8, dadurch gekennzeichnet, dass man als wässerige Flüssigkeit Blut verwendet.

10. Verfahren zur Prüfung auf oder Bestimmung von Bilirubin ($B_u$, $B_c$ oder $B_T$) in einer wässerigen Flüssigkeit, gekennzeichnet durch die Stufen:

(a) Behandlung einer Probe der Flüssigkeit mit einem Bilirubin-spezifischen Enzym nach Anspruch 1 zum Zwecke der Reaktion von Bilirubin mit dem Enzym und zur Erzeugung einer feststellbaren Änderung und

(b) Bestimmung der in Stufe (a) erzeugten Änderung.

11. Verfahren zur Prüfung auf oder Bestimmung eines von Bilirubin verschiedenen Analyten in einer wässerigen Flüssigkeit, die Bilirubin enthält und in der Bilirubin störend wirkt, gekennzeichnet durch die Stufen:

(a) Behandlung einer probe der Flüssigkeit mit einem reaktiven Reagens für den Analyten zur Erzeugung einer feststellbaren Veränderung,

(b) Behandlung der Probe vor oder während der Stufe (a) mit einem Bilirubin-spezifischen Enzym nach Anspruch 1 unter Abbau des Bilirubins und Verminderung seines Störpotentials bezüglich der in Stufe (a) erzeugten bestimmbaren Veränderung und

(c) Bestimmung der in Stufe (a) erzeugten Veränderung.

12. Verfahren nach Anspruch 10 oder 11, dadurch gekennzeichnet, dass das Enzym oder das reaktive Reagens in einem trockenen analytischen Element nach einem der Ansprüche 4 bis 7 enthalten ist.

13. Die Erfindung nach einem der Ansprüche 1-12, dadurch gekennzeichnet, dass das Enzym von einer Pflanze der Gattung *Solanum, Capsicum* oder *Lycopersicum* aus der Familie der Solanaceaen stammt.

14. Die Erfindung nach einem der Ansprüche 1 bis 12, dadurch gekennzeichnet, dass das Enzym von einer Pflanze der Gattung *Musa* aus der Familie der Musaceaen stammt.

15. Die Erfindung nach einem der Ansprüche 1 bis 12, dadurch gekennzeichnet, dass das Enzym von einer Pflanze der Gattung *Allium* aus der Familie der Liliaceaen stammt.

16. Die Erfindung nach einem der Ansprüche 13 bis 15, dadurch gekennzeichnet, dass das Enzym von *Solanum melongena, Musa sapientum, Allium cepa* oder *Allium satiuum* stammt.

## Revendications

1. Enzyme spécifique de la bilirubine, caractérisée en ce qu'elle provient de plantes appartenant aux familles des Solanacées, des Musacées et des Liliacées qui, en présence d'un liquide aqueux contenant de la bilirubine, dégrade la bilirubine ($B_u$) à un pH de 6 à 10, la bilirubine ($B_c$) à un pH de 2 à 11 et les bilirubines $B_u$ et $B_c$ à une température de 20 à 50° C.

2. Réactif analytique comprenant un réactif interactif pour le dosage ou la détection d'un constituant autre que la bilirubine dans un échantillon de liquide aqueux, caractérisé en ce qu'il comprend une enzyme spécifique de la bilirubine provenant d'une plante d'un groupe de familles consistant dans les Solanacées, les Musacées et les Liliacées qui, en présence d'un liquide aqueux contenant de la bilirubine, dégrade la bilirubine ($B_u$) à un pH de 6 à 10, la bilirubine ($B_c$) à un pH de 2 à 11 et les bilirubines ($B_u$) et ($B_c$) à une température de 20 à 50° C.

3. Réactif analytique selon la revendication 2, qui contient un tampon pour maintenir le pH dans l'intervalle 2 à 11.

4. Produit d'analyse par voie sèche, pour le dosage ou la détection d'un constituant, caractérisé en ce qu'il comprend une enzyme selon la revendication 1 ou un réactif analytique selon les revendications 2 ou 3.

5. Produit selon la revendication 4, comprenant un support et une zone de réactif contenant l'enzyme ou le réactif analytique.

6. Produit selon la revendication 4 ou 5, comprenant une zone d'étalement absorbante.

7. Produit selon l'une des revendications 4 à 6, dans lequel l'enzyme est présente à raison de 40 à 400 U/m².

8. Procédé pour la dégradation de la bilirubine dans un liquide aqueux, procédé qui est caractérisé en ce qu'on traite le liquide avec une enzyme spécifique de la bilirubine conforme à la revendication 1 pour dégrader la bilirubine.

9. Procédé selon la revendication 8, dans lequel le liquide aqueux est le sang complet.

10. Procédé pour le dosage ou la détection de la bilirubine ($B_u$, $B_c$, ou $B_T$) dans un liquide aqueux, procédé caractérisé par les étapes suivantes:

(a) on traite un échantillon du liquide avec une enzyme spécifique de la bilirubine selon la revendication 1 pour faire interagir la bilirubine avec l'enzyme et pour produire un changement détectable, et

(b) on détecte le changement produit dans l'étape (a).

11. Procédé pour le dosage ou la détection d'un constituant autre que la bilirubine dans un liquide aqueux contenant de la bilirubine, laquelle constitue un interférent, ce procédé étant caractérisé par les étapes suivantes:

(a) on traite un échantillon du liquide avec un réactif interactif pour que le constituant produise un changement détectable,

(b) avant ou pendant l'étape (a), on traite l'échantillon avec une enzyme spécifique de la bilirubine selon la revendication 1, et ainsi on dégrade la bilirubine et l'on réduit sa capacité à interférer avec le changement détectable produit dans l'étape (a) et

(c) on détecte le changement produit dans l'étape (a).

12. Procédé selon la revendication 10 ou 11 dans lequel l'enzyme ou le réactif interactif est contenu dans un produit pour analyse par voie sèche selon l'une des revendications 4 à 7.

13. L'invention selon l'une des revendications 1 à 12, dans laquelle l'enzyme provient d'une plante du genre *Solanum, Capsicum* ou *Lycopersicum* de la famille des Solanacées.

14. L'invention selon l'une des revendications 1 à 12, dans laquelle l'enzyme provient d'une plante du genre *Musa* de la famille des Musacées.

15. L'invention selon l'une des revendications 1 à 12 dans laquelle l'enzyme provient d'une plante du genre *Allium* de la famille des Liliacées.

16. L'invention selon l'une des revendications 13 à 15 dans laquelle l'enzyme provient de *Solanum melongena, Musa sapientum, Allium cepa* ou *Allium sativum.*